# EUROPEAN PATENT APPLICATION

(11) **EP 2 431 717 A1**
(43) Date of publication of application: **21.03.2012**
(21) Application number: 09839605.4
(22) Date of filing: 28.11.2009
(51) Int. Cl.: G01G 19/44

(54) **BODY WEIGHT MANAGEMENT DEVICE, BODY WEIGHT MANAGEMENT METHOD, AND BODY WEIGHT MANAGEMENT PROGRAM**

(30) Priority: 09.02.2009 JP 2009027502
(71) Applicant: Omron Healthcare Co., Ltd., Ukyo-ku Kyoto-shi Kyoto 615-0084 (JP)
(72) Inventor: OSHIMA, Yoshitake, Kyoto-shi Kyoto 615-0084 (JP); DOI, Ryosuke, Kyoto-shi Kyoto 615-0084 (JP)
(74) Representative: Vossius & Partner
(86) International application number: PCT/JP2009/006453
(87) International publication number: WO 2010/089827

(57) **Abstract**

To provide a weight management device enabling the user to check the daily target indicating to what extent the weight fluctuation in the current day is to be maintained, so that the user can carry out the management of the daily weight fluctuation according to the plan, and the satisfaction level of the user can be enhanced.

A weight and body composition meter 3 is configured to acquire a weight measurement value of a user with a load detection unit 33, calculate a standard daily fluctuation amount data 41 indicating a standard daily fluctuation of the weight of the user, and calculate a daily target increase and decrease amount data 45 to become a target when measuring a night weight at night with respect to a morning weight measured in the morning from when the user wakes up until going to bed with a control unit 18 based on the standard daily fluctuation amount data 41.

## Description

### TECHNICAL FIELD

The present invention relates to a weight management device, a weight management method, and a weight management program for managing the weight of the user that fluctuates daily.

### BACKGROUND ART

Conventionally, the weight is with a weigh whether the weight is increasing or decreasing is checked. Here, the weigh is known to have daily fluctuation of increasing from the time of waking up until the time of going to bed, and decreasing from the time of going to bed until the time of waking up. Thus, the influence of the daily fluctuation may be received if the measurement time is different, and whether the weight is increasing or decreasing may not be accurately known.

With respect to this problem, a health condition determining device for determining the health condition by removing the daily fluctuation from the measurement weight is proposed (see patent document 1). This health condition determining device sets a reference value based on a measurement value measured at substantially the same time of the weight values in a plurality of past days in which the measurement of the weight value of a plurality of times is carried out within one day, and compares the reference value and the current value to determine whether the weight is in the weight decreasing tendency.

A biological measurement device with graph display function is also proposed (see patent document 2). The biological measurement device is devised so that the user can easily understand the history of the measurement results when displaying the past weight measurement results in time series.

However, both devices merely grasp the past background and the current state, and a short-term target cannot be set based on the measurement result.

Patent Document 1: Japanese Unexamined Patent Publication No. 2005-218582
Patent Document 2: Japanese Patent Publication No. 3818488

### DISCLOSURE OF THE INVENTION

### PROBLEMS TO BE SOLVED BY THE INVENTION

In view of the above problems, it is an object of the present invention to provide a weight management device enabling the user to check the daily target indicating to what extent the weight fluctuation in the current day is to be maintained, so that the user can carry out the management of the daily weight fluctuation according to the plan, and the satisfaction level of the user can be enhanced.

### MEANS FOR SOLVING THE PROBLEM

The present invention relates to a weight management device including weight acquiring means for acquiring a weight measurement value of a user, standard daily fluctuation amount acquiring means for acquiring standard daily fluctuation amount indicating a standard daily fluctuation of the weight of the user, and calculation means for calculating a daily target value that becomes a target when measuring a going to bed side weight measurement value at a second timing on the going to bed side with respect to a waking up side weight measurement value measured at a first timing on a waking up side from waking up until going to bed of the user based on the standard daily fluctuation amount.

The weight acquiring means may be configured with an appropriate device for acquiring weight such as being configured with a load detection device such as a load detection unit including a load cell or being configured with a communication device for receiving weight measurement value data.

The standard daily fluctuation amount may be an appropriate value such as sleep weight fluctuation amount based on the weight fluctuation amount from the going to bed side weight measurement value to the waking up side weight measurement value, the daily fluctuation amount obtained from the basal metabolism amount, or a predefined constant value.

The standard daily fluctuation amount acquiring means may be configured by appropriate means such as calculation means for acquiring the weight fluctuation amount from the night weight to the morning weight over plural days and calculating an average value of the plurality of weight fluctuation amounts, calculation means for calculating a basal metabolism from the physical information and the impedance of the user and calculating the daily fluctuation amount, or information processing means for reading out a predefined constant value from the storage unit.

The first timing is timing within a time obtained by adding a predetermined time to the time zone in which a typical person is likely to wake up, and is preferably from waking up until breakfast.

The second timing is a timing other than the first timing or a timing measured within a time obtained by adding a predetermined time to the time zone in which a typical person is likely to go to bed, and is preferably from dinner until going to bed.

The daily target value may be a value with which to what extent of the measurement value the weight at the time of going to bed is to be set with respect to the weight at the time of waking up in a day can be recognized such as the target weight value at the time of going to bed or the target value of the difference between the weight at the time of waking up and the weight at the time of going to bed.

The weight management device can be configured with a weight scale for measuring weight, a weight and body composition meter for measuring weight and body composition, or an information processing device for acquiring information from the weight scale and the weight and body composition meter. The information processing device may be a portable or a desktop device for performing information processing such as a personal computer, a PDA (Personal Digital Assistants), or a portable telephone.

According to the present invention, the user can check the daily target indicating to what extent the daily weight fluctuation is to be maintained. Therefore, the user can manage the daily weight fluctuation according to the plan.

In one aspect of the present invention, output means for outputting the daily target value is arranged, where the calculation means outputs thy daily target value to the output means when acquiring the waking up side weight measurement value from the weight acquiring means.

The output means may be configured by means for outputting the daily target value such as a display device for displaying images such as characters, numbers, and figures, a light emitting device such as an LED or a lamp that emits light, or a communication device for transmitting data.
According to the aspect, the daily target value can be output.

In another aspect of the present invention, storage means for storing the waking up side weight measurement value is arranged, where the calculation means acquires the going to bed side weight measurement value from the weight acquiring means, executes a target attainment determination process of determining whether or not the daily target is attained based on the going to bed side weight measurement value, the daily target value, and the waking up side weight measurement value, and outputs the determination result of the target attainment determination process to the output means.

The target attainment determination process may be a determination process related to attaining the target such as determining whether or not the increase of greater than or equal to the target value is obtained if the determination is for increasing weight, determining whether or not the decrease of greater than or equal to the target value is obtained if the determination is for decreasing weight, or determining whether or not the weight is the same as the target value or within a predetermined value from the target value if determination is for maintaining the weight.

The determination result may be an appropriate result such as a result of whether or not the target value attained, or result of what extent the attainment degree is.
According to the aspect, the user can clearly recognize whether or not the target is attained.

In another aspect of the present invention, the calculation means may calculate the standard daily fluctuation amount based on a plurality of sleep weight fluctuation amount that is the difference between the going to bed side weight measurement value measured over plural days in the past and the waking up side weight measurement value of the next day.

The sleep weight fluctuation amount can be calculated based on a plurality of sleep weight fluctuation amounts such as average value, median value, or most frequent value of the plurality of sleep weight fluctuation amounts. This calculation can be performed using a predetermined previous number (e.g., for one week or for two weeks) of sleep weight fluctuation amounts for every measurement or performed using all the sleep weight fluctuation amounts.

According to the aspect, the standard daily fluctuation amount can be calculated based on the weight fluctuation of the user that is actually measured. In particular, since the standard daily fluctuation amount is calculated using the weight fluctuation during sleep, the standard daily fluctuation amount can be obtained even if the weight is in the increasing tendency or the decreasing tendency without being influenced by the tendency. In other words, if calculated using the weight fluctuation amount from the waking up side weight measurement value to the going to bed side weight measurement value of the current day, the large fluctuation amount by the increased amount of weight from the original daily fluctuation amount may become the standard daily fluctuation amount if the weight is in the increasing tendency, but this can be prevented by using the weight fluctuation during sleep that is not influenced by the increasing or decreasing tendency of the weight.

In another aspect of the present invention, basal metabolism amount acquiring means for acquiring the basal metabolism amount of the user based on the physical information related to the body of the user and the impedance of the body of the user, where the calculation means is configured to calculate the standard daily fluctuation amount based on the basal metabolism amount.

The basal metabolism amount acquiring may be configured by appropriate means such as being configured by physical information acquiring means for acquiring the physical information related to the body of the user, impedance acquiring means for acquiring the impedance of the body of the user, and calculation means for calculating the basal metabolism amount of the user based on the physical information and the impedance, or being configured by communication means for receiving the basal metabolism amount information.

The physical information may be configured by sex, age, and height of the user, or weight in addition to the above.
The physical information acquiring means may be configured by operation input means for receiving the operation input of the user, communication means for receiving the physical information, measurement means for measuring weight or the like, or a plurality of the same.

The impedance acquiring means may be configured by means for acquiring the impedance such as impedance detection means for detecting the impedance by flowing current to the user from the electrode.

According to the aspect, the standard daily fluctuation amount can be calculated based on the basal metabolism amount. Therefore, even if the weight is in increasing tendency or in decreasing tendency, the standard daily fluctuation amount can be obtained without being influenced by the tendency.

In another aspect of the present invention, the calculation means may have a configuration to execute the waking up side weight measurement value updating process of updating the waking up side weight measurement value to the remeasured weight measurement value when the weight is remeasured within a predetermined time from the first timing at which the waking up side weight measurement value is measured and such remeasured weight measurement value is lower than the waking up side weight measurement value.

The predetermined time from the first timing may be an appropriately defined time such as 30 minutes or 1 hour. The predetermined time is preferably set to a time the possibility of weight loss due to exercise is low.

The waking up side weight measurement value updating process may be a process of storing so that the final waking up side weight measurement value can be recognized such as a process of overwriting and updating the already stored waking up side weight measurement value to the remeasured weight measurement value or a process of storing both the already stored waking up side weight measurement value and the remeasured weight measurement value and providing flag or information indicating the waking up side weight measurement value to the remeasured weight measurement value.

According to the aspect, when the user goes to the restroom after the measuring the waking up side weight and then remeasures, the weight decreased by going to the restroom may be set as the waking up side weight measurement value. Therefore, the weight after going to the restroom may be set as the waking up side weight measurement value, so that the waking up side weight measurement value can be stabilized. Moreover, since the updatable remeasurement is limited to a predetermined time, the possibility the remeasurement is carried out after the weight is decreased by exercise after the measurement can be reduced.

In another further aspect of the present invention, the calculation means may be configured to execute a going to bed side weight measurement value determination process of adopting the weight measurement value measured the last within a predetermined time as the going to bed side weight measurement value when the weight is measured over plural times within the predetermined time of recognized as the going to bed side weight measurement value.

The predetermined time recognized as the going to bed side weight measurement value may be appropriately defined to a time other than the time range of being recognized as the waking up side weight measurement value or a time assumed as after dinner. The predetermined time is preferably a time that does not overlap the time range recognized as the waking up side weight measurement value.

The going to bed side weight measurement value determination process may be a process of storing so that the final going to bed side weight measurement value can be recognized such as a process of overwriting and updating the already stored going to bed side weight measurement value to the remeasured weight measurement value or a process of storing both the already stored going to bed side weight measurement value and the remeasured weight measurement value and providing flag or information indicating the going to bed side weight measurement value to the remeasured weight measurement value.

According to the aspect, the final weight measurement value before going to bed can be set as the going to bed side weight measurement value even if the user performs the weight measurement over plural times in one day. Therefore, the user can measure the weight at any time without being conscious of the value being stored as the going to bed side weight measurement value, and thus use can be made without affecting the convenience.

In another further aspect of the present invention, the calculation means can calculate the daily target increase and decrease amount by dividing the long term target increase and decrease amount that is the target weight increase and decrease amount by the target number of days until attaining the target, and define the daily target value based on the daily target increase and decrease amount and the standard daily fluctuation amount.

The long term target increase and decrease amount may be the increase and decrease amount input by the operation input means or the increase and decrease amount defined in advance. The input by the operation input means may be received through an appropriate method such as numerical input or selection input from plural candidates.

The target number of days may be number of days input by the operation input means or the number of days defined in advance. The input by the operation input means may be received through an appropriate method such as numerical input or selection input from plural candidates.

According to the aspect, the user can set a long term increase and decrease target, and grasp to what extent to have the weight increase and decrease amount of one day to attain the long term increase and decrease target. Therefore, when dieting, the weight can be managed while recognizing attainment/no attainment of the target in units of one day, so that the weight can be finely managed to attain the long term target.

In another aspect of the present invention, the first timing is from when the user wakes up until before breakfast, and the second timing is from after dinner until before the user goes to bed.

Therefore, the waking up side weight measurement value and the going to bed side weight measurement value are stably and accurately acquired. To easily understand this, the input device and the display device may be used to selectively input the measurement of after waking up until breakfast, the measurement of after dinner until before going to bed, and the like.

The present invention relates to a weight management device including weight acquiring means for acquiring a weight measurement value of a user, information processing means for performing information processing, and display means for performing display of the screen, where the information processing means displays on the display means the waking up side weight display screen if the weight measurement value of the user acquired by the weight acquiring means is the waking up side weight measurement value measured at the first timing on the waking up side from when the user wakes up until going to bed, and displays on the display means the going to bed side weight display screen different from the waking up side weight display screen if the weight measurement value is the going to bed side weight measurement value measured at the second timing on the going to bed side.

The information processing means may be configured to execute a timing determination process of determining the measurement timing of the weight measurement value of the user. The timing determination process determines either as the first timing or the second timing or determines as timing other than the first timing or the second timing.

The waking up side weight display screen may be configured to include one of (1) to (7) below.
(1) Measurement weight value of first timing of current day, and target weight value of second timing of current day.
(2) Measurement weight value of first timing of current day, and target value of difference in weight from the time of waking up until the time of going to bed of current day.
(3) Measurement weight value of first timing of current day, and measurement weight value of first timing of previous day.
(4) Measurement weight value of first timing of current day, and weight decrease value from second timing of previous day to first timing of current day.
(5) Measurement weight value of first timing of current day, weight decrease value from second timing of previous day to first timing of current day, and weight increase value from first timing of previous day to second timing of previous day.
(6) Measurement weight value of first timing of current day, weight decrease value from second timing of previous day to first timing of current day, and target value of difference in weight from first timing to second timing of current day.
(7) Graph (line graph, bar graph, etc.) or meter indicating the increasing or decreasing tendency of the measurement weight value of the first timing and the measurement weight value of the second timing.

The going to bed side weight display screen can be configured to include one of (1) to (7) below.
(1) Measurement weight value of second timing of current day, and target weight value of first timing of current day.
(2) Measurement weight value of second timing of current day, and attainment check display indicating whether or not target of current day is attained (attainment check display may be an appropriate display such as graph or meter, or attainment possibility display).
(3) Measurement weight value of second timing of current day, and measurement weight value of second timing of previous day.
(4) Measurement weight value of second timing of current day, and weight increase value from first timing of current day to second timing of current day.
(5) Measurement weight value of second timing of current day, weight increase value from first timing of current day to second timing of current day, and weight reduction value from second timing of previous day to first timing of current day.
(6) Measurement weight value of second timing of current day, weight increase value from first timing of current day to second timing of current day, and attainment check display indicating whether or not target of current day is attained.
(7) Graph (line graph, bar graph, etc.) or meter indicating the increasing or decreasing tendency of the measurement weight value of the first timing and the measurement weight value of the second timing.

According to the present invention, the user can check the target of the current day with the weight measurement value of the first timing on the waking up side, and clearly check whether or not the target is attained with the weight measurement value of the second timing on the going to bed side. In particular, the convenience of weight management can be enhanced by clearly showing the weight increasing or decreasing tendency for the weight management of the living boy in which weight increasing or decreasing tendency of a short period of time is difficult to grasp due to daily fluctuation by differing the display content according to the measurement timing. Furthermore, the user can carry out an appropriate comparison with the measurement weight value of the previous day, the comparison of the measurement weight values within the current day, the recognition of the target, or the check on whether or not the target is attained by each display content of the waking up side weight display screen and the going to bed side weight display screen.

The present invention relates to a weight management method including the steps of acquiring a weight measurement value of a user with weight acquiring means, acquiring a standard daily fluctuation amount indicating a standard daily fluctuation of the weight of the user with standard daily fluctuation amount acquiring means, and calculating a daily target value to become a target when measuring a going to bed side weight measurement value at a second timing on the going to bed side with respect to a waking up side weight measurement value measured at a first timing on the waking up side from when the user wakes up until going to bed based on the standard daily fluctuation amount with calculation means.

The user can check the daily target indicating to what extent the daily weight fluctuation is to be maintained. Therefore, the user can manage the daily weight fluctuation according to the plan.

The present invention relates to a weight measurement program for causing a computer to function as weight acquiring means for acquiring a weight measurement value of a user, standard daily fluctuation amount acquiring means for acquiring a standard daily fluctuation amount indicating a standard daily fluctuation of the weight of the user, and calculation means for calculating a daily target value to become a target when measuring a going to bed side weight measurement value at a second timing on the going to bed side with respect to a waking up side weight measurement value measured at a first timing on the waking up side from when the user wakes up until going to bed based on the standard daily fluctuation amount.

The user can check the daily target indicating to what extent the daily weight fluctuation is to be maintained by simply installing the weight management program in the computer. Therefore, the user can manage the daily weight fluctuation according to the plan.

### EFFECT OF THE INVENTION

According to the present invention, the user can check the daily target indicating to what extent the daily increase weight is to be maintained, and can manage the daily weight fluctuation according to the plan. The satisfaction level of the user thus can be enhanced.

### BRIEF DESCRIPTION OF THE DRAWINGS

Fig. 1 is a perspective view showing an outer appearance of a weight and body composition meter.
Fig. 2 is a block diagram showing a configuration of a weight and body composition meter and a server.
Figs. 3A to 3G are configuration diagrams of various types of data stored in the storage unit.
Fig. 4 is a flowchart showing the operation executed by a control unit of the body composition meter.
Fig. 5 is a flowchart showing the operation of the storing/analyzing process.
Figs. 6A to 6D are explanatory views of the display screens describing the screen displayed on the display unit.
Figs. 7A and 7B are explanatory views describing the daily fluctuation of the weight.

### BEST MODE FOR CARRYING OUT THE INVENTION

One embodiment of the present invention will be described below with reference to the drawings.

### [Examples]

In the example, an example of a body composition meter capable of measuring not only the weight but also the body fat percentage will be described.

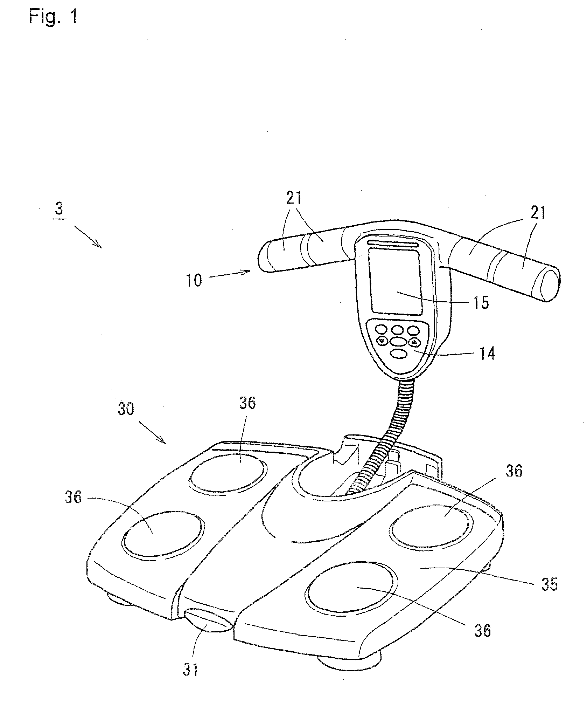

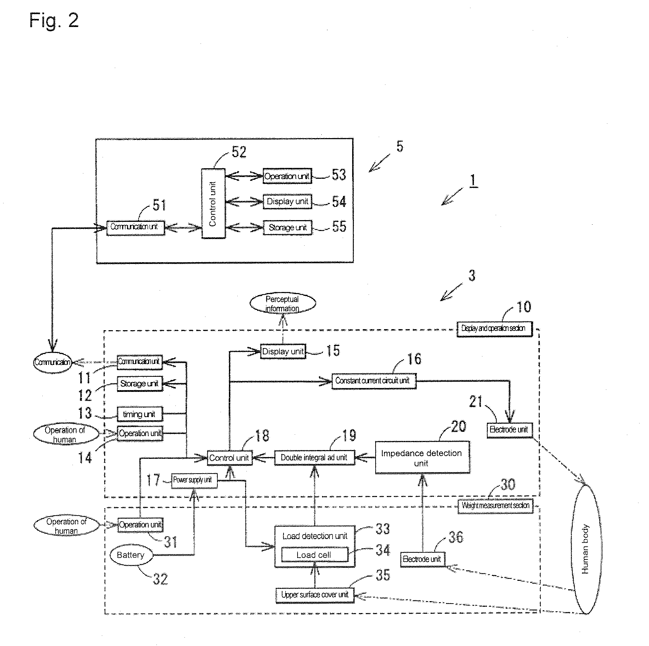

Fig. 1 is a perspective view showing an outer appearance of a weight and body composition meter 3, and Fig. 2 is a block diagram showing a configuration of a weight management system 1 configured by the weight and body composition meter 3 and a server 5 capable of communicating with the weight and body composition meter 3.

As shown in Fig. 1, the weight and body composition meter 3 is mainly configured by a display and operation section 10, which is a first housing to be held by the user with hand, and a weight measurement section 30, which is a second housing on which the user goes on.

As shown in Fig. 2, the display and operation section 10 includes a communication unit 11, a storage unit 12, a timing unit 13, an operation unit 14, a display unit 15, a constant current circuit unit 16, a power supply unit 17, a control unit 18, a double integral AD unit 19, an impedance detection unit 20, and an electrode unit 21.

The communication unit 11 is connected to the control unit 18, and communicates with the server 5 according to a control signal of the control unit 18. The communication unit 11 may be configured to communicate not only with the server 5 but also with an appropriate device such as communicate with other biological information acquiring devices including a pedometer or communicate with a personal computer or a personal information terminal (PDA or portable telephone, etc.).

The storage unit 12 is configured by a device capable of storing information such as nonvolatile memory and hard disc, and carries out read and write of information according to the control signal of the connected control unit 118.

The timing unit 113 (timing means) is a device for timing the time such as the current date and time, and transmits the time to the control unit 18 as needed.
The operation unit 14 is configured by a plurality of buttons (see Fig. 1) to be push-operated, and the input information push-operated by the user such as input of physical information of the user including sex, age, height, and weight is transmitted to the control unit 18.

The display unit 15 is configured by a display device such as a liquid crystal screen (see Fig. 1), and displays characters and figures according to an image signal transmitted from the control unit 18.
The constant current circuit unit 16 flows high frequency (AC) current supplied from the power supply unit 17 in one direction to the electrode unit 21 for current application based on the control of the control unit 18.

The power supply unit 17 supplies the operation power to each unit including the control unit 18.
The control unit 18 is configured by a CPU, a ROM, a RAM, or a microcomputer, and executes the control operation and the calculation operation of each unit according to a program stored in the ROM, or the like. The weight management program is stored for the program.

The double integral AD unit 10 is a double integral type AD (Analog/Digital) converter, and converts an analog signal provided from the impedance detection unit 20 to a digital signal.

The impedance detection unit 20 detects the impedance of the user based on a potential difference of the electrode unit 36 arranged in the weight measurement section 30 and the electrode unit 21 arranged in the display and operation section 10.

The electrode unit 21 is arranged on the surface of the grip portion (see Fig. 1) of the display and operation section 10 which the user holds with his/her hand, and applies high frequency (AC) current supplied from the power supply unit 17 to the palm of the user gripping the grip portion.

The weight measurement section 30 is configured by an operation unit 31, a battery 32, a load detection unit 33, and an electrode unit 36.
The operation unit 31 functions as an input switch for switching ON/OFF of the power supply, and transmits the inputted input signal to the control unit 18.

The buttery 32 supplies power to each unit with the power supply unit 17 as the center.
The load detection unit 33 incorporates a load cell 34, and measures the weight of the user who got on an upper surface cover unit 35 (see Fig. 1) also serving as an upper surface cover of the housing. The weight measured here is transmitted to the double integral AD unit 19.

The electrode unit 36 is arranged on the surface of the upper surface portion (see Fig. 1) of the weight measurement section 30 on which the user goes on, and is an electrode for current measurement that receives the current flowing from the back of the foot of the user. The electrode unit 36 is configured by four electrodes, that is, the electrodes of the left toe side, left heel side, right toe side, and right heel side of the user.

The server 5 is configured by a communication unit 51, a control unit 52, an operation unit 53, a display unit 54, and a storage unit 55.
The communication unit 51 performs transmission and reception of data with the weight and body composition meter 3 according to the control of the control unit 52.
The control unit 52 is configured by a CPU, a ROM, and a RAM, and executes the control operation and the calculation operation of each unit according to a program stored in the ROM, or the like.

The operation unit 53 is configured by an operation input device such as a keyboard or a mouse, and transmits the operationally inputted input signal to the control unit 52.
The display unit 54 is configured by a display device such as a liquid crystal display and a CRT display, and carries out the display according to a control signal of the control unit 52.

The storage unit 55 is configured by a storage device such as a hard disc, and stores various data related to the user such as the body composition data (body fat data and weight data) measured with the weight and body composition meter 3 and personal information such as name and address of the user.
The weight management system 1 is configured by the weight and body composition meter 3 and the server 5 configured as above.

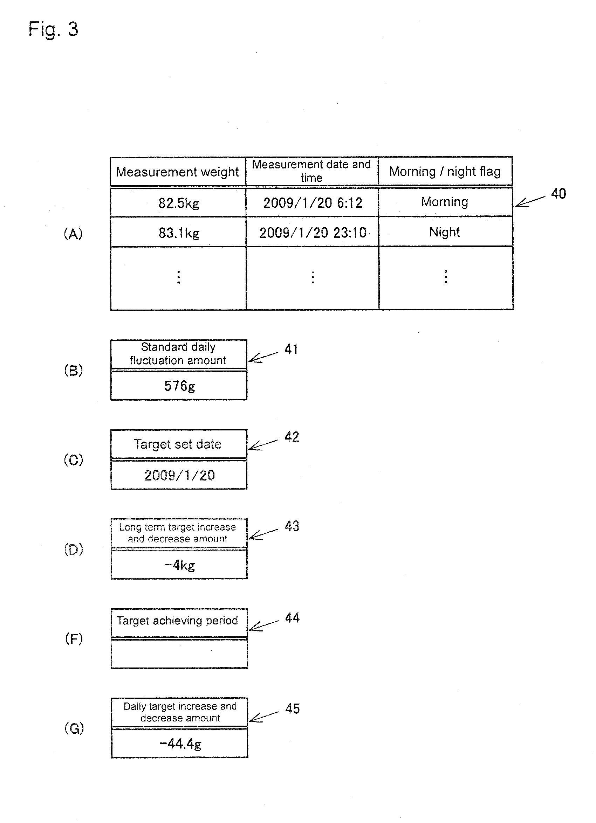

Figs. 3A to 3G are configuration diagrams of various types of data stored in the storage unit 12.
The measurement data 40 shown in Fig. 3A stores the measurement weight with the measurement date and time. This measurement data 40 also stores a morning/night flag indicating whether the weight is the morning weight measured in the morning (waking up) or the night weight measured at night (going to bed). The measurement data 40 may be stored for one week or for two weeks, and may be configured to store for a longer period time.

A standard daily fluctuation amount data 41 shown in Fig. 3B stores the standard daily fluctuation amount of the user. The standard daily fluctuation amount is calculated based on the past measurement data 40 (for one week or for two weeks). This calculation may be carried out in the following manor.

In other words, the sleep weight fluctuation amount indicating the weight that fluctuated during sleep is calculated by subtracting the morning weight of the next day from the night weight, and the sleep weight fluctuation amount is calculated for a plurality of days to calculate the average value thereof, the average value becoming the standard daily fluctuation amount.

Here, with respect to one day, one day is set as from the time of waking up until the time of going to bed of the user. Therefore, even if the user measures the night weight after 24 o'clock, in a precise sense, and goes to bed, it is not set as the weight night of the next day and is preferably handled as the night weight of the same day.

Another method of calculating the standard daily fluctuation amount uses a basal metabolism amount obtained from the impedance of the user detected by the impedance detection unit 20. In this case, the body composition such as the basal metabolism amount is calculated through a known method based on the detected impedance, the height, age, and sex of the user appropriately stored in the storage unit 12, and the weight detected by the load detection unit 33. The body composition calculated in this case is not limited to the basal metabolism amount, and may include the body fat percentage, the BMI, the visceral fat level, the skeletal muscle percentage, and the body age. The sleep weight fluctuation amount in which the calorie is consumed during sleep and the weight fluctuates can be calculated through the known method based on the calculated basal metabolism amount, and such sleep weight fluctuation amount can be set as the standard daily fluctuation amount.

The standard daily fluctuation amount assuming a state in which the weight is stable without increasing or decreasing can be obtained even if the weight of the user is in the increasing tendency or the decreasing tendency by calculating the standard daily fluctuation amount through such methods.

The standard daily fluctuation amount may have a simple configuration of being fixed to a daily fluctuation amount of a general adult defined in advance.

The target set date data 42 shown in Fig. 3C stores the year, month, and day on which the user input and set the target of the weight increase or decrease with the operation unit 14.

The long term target increase and decrease amount data 43 shown in Fig. 3D stores the target value of the weight increase or decrease amount input-set by the user with the operation unit 14. For instance, a negative value is input-set when desiring to lose weight through diet, 0 is input-set when desiring to maintain weight, and a positive value is input-set when desiring to increase weight.

The target achieving period data 44 shown in Fig. 3F stores the target achieving period input-set by the user with the operation unit 14. The target achieving period is the period indicating in what period the long term target increase and decrease amount is desirably achieved. When the target achieving period is input-set, an appropriate determination on whether or not the daily increase and decrease amount that becomes the norm of one day in which the long term target increase and decrease amount is divided by the number of days of the target achieving period is within a predetermined range is to be executed. If determined as outside the predetermined range in the appropriate determination, a process of error display on the display unit 15 or prohibiting registration may be carried out. The stiff diet or the like that imposes burden on the body can be suppressed.

The daily target increase and decrease amount data 45 shown in Fig. 3G is set with a value obtained by dividing the standard weight increase and decrease amount by the target achieving period. By the daily target increase and decrease amount data 45, whether or not the increase or the decrease of the weight is progressing on the target can be checked in unit of one day, and the target weight at night of the current day can be calculated.

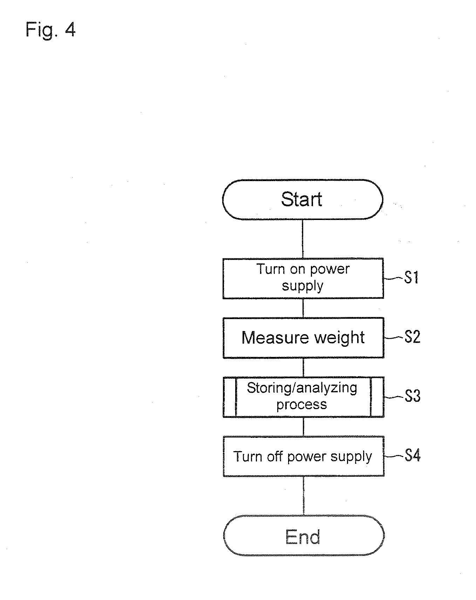

Fig. 4 is a flowchart showing the operation executed by the control unit 18 of the weight and body composition meter 3.
The control unit 18 is started up when the power supply is turned ON by the user (step S1), and measures the weight of the user that got on the upper surface cover unit 35 (see Fig. 1) with the load detection unit 33 (step S2). In this case, the control unit 18 also executes the measurement of the body composition by the impedance detection unit 20 (see Fig. 2) through the electrode unit 36 arranged in the weight measurement section 30 and the electrode unit 21 arranged in the display and operation section 10.

The control unit 18 executes the storing / analyzing process (step S3), turns OFF the power supply (step S4), and terminates the process.

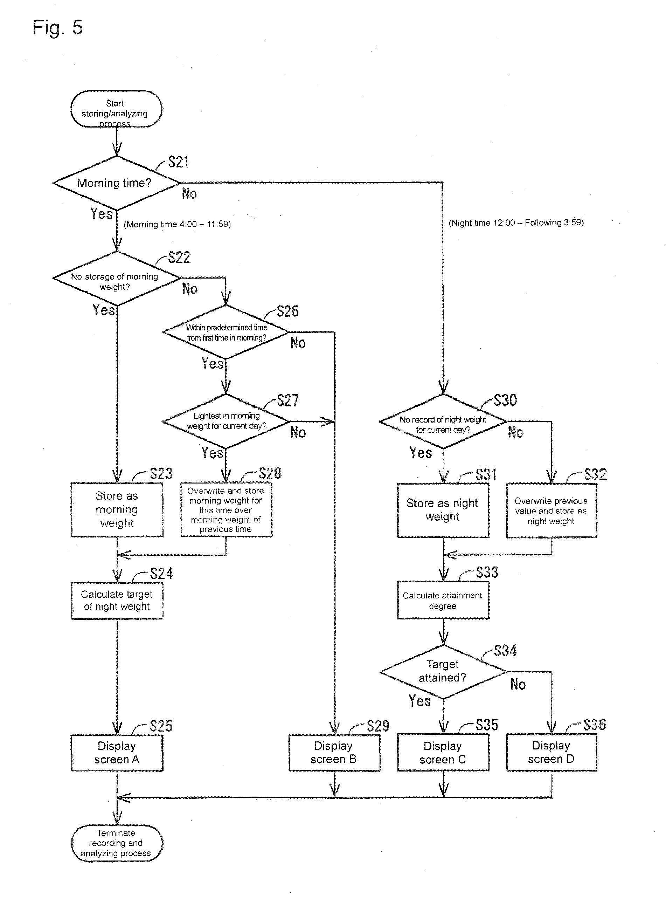

Fig. 5 is a flowchart showing the operation of the storing/analyzing process executed by the control unit 18 of the weight and body composition meter 3.
The control unit 18 acquires the current date and time that is the date and time, in which the weight is measured in step S2, from the timing unit 13, and determines whether or not the current date and time corresponds to the morning time (step S21). In this example, the morning time is from 4 AM to 11:59AM and the night time is from 12PM to 3:59AM the next morning, that is, the other time.

If in the morning time (step S21: Yes), the control unit 18 checks whether or not the morning weight for today is already stored by referencing the storage unit 12 (step S22). This check is executed through an appropriate method such as determining whether or not the morning weight from the measurement date and time by adopting a configuration of storing the measurement date and time with the weight, determining with reference to the morning weight flag by adopting a configuration of storing the morning weight flag indicating whether or not the morning weight with the weight, or defining the address of the storage nit 12 for storing the morning weight and determining the weight stored in the relevant address as the morning weight.

If the morning weight is not stored (step S22: Yes), the control unit 18 stores the weight measured in step S2 in the storage unit 12 as the morning weight (step S23). In this case, the measurement date and time is also stored. The morning weight flag indicating whether or not the stored weight is the morning weight may also be stored or the address of the storage unit 12 for storing the weight as the morning weight may be defined and the weight may be stored in such address.

The control unit 18 calculates the target of the night weight (step S24). The measurement weight stored in the storage unit 12 as the morning weight and the standard daily fluctuation amount data 41 and the daily target increase and decrease amount data 45 stored in the storage unit 12 are read out, and the calculation of the target is executed based thereon. The target daily fluctuation amount indicating to about how much grams to realize for the weight fluctuation amount of one day or the target night weight indicating to about how much kilograms to realize for the night weight.

The target daily fluctuation amount can be obtained by adding the daily target increase and decrease amount of Fig. 3G to the standard daily fluctuation amount of Fig. 3B.
The target night weight can be obtained by adding the standard daily fluctuation amount of Fig. 3B and the daily target increase and decrease amount of Fig. 3G to the morning weight.

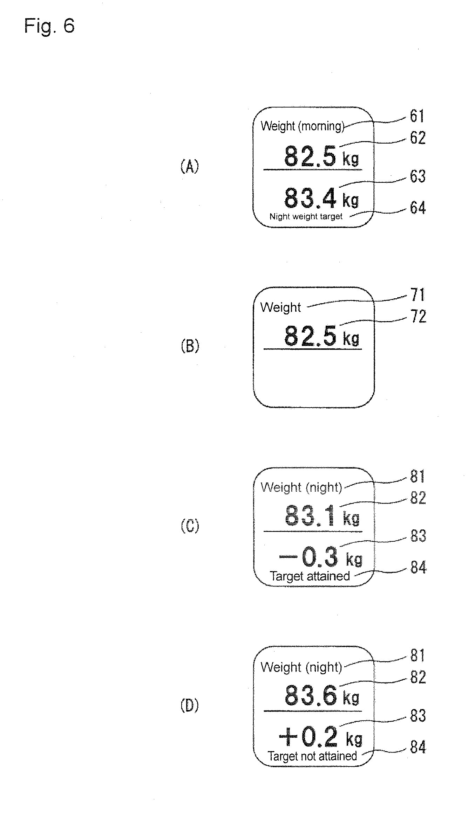

As shown in Fig. 6A, the control unit 18 displays a morning measurement display screen A inducing the ca!culated night weight target and the measurement weight measured in stop S2 (step S25). The morning measurement display screen A includes a morning weight title display 61 indicating that the weight is the morning weight, a measurement weight display 62 indicating the measurement value of the morning weight, a target display 63 indicating the target value of the night weight, and a target title display 64 indicating a target value of the night weight. In this example, the target night weight is displayed as the target of the night weight.

If the morning weight of today is stored in the storage unit 12 in step S22 (step S22: No), the control unit 18 determines whether or not within a predetermined time from the first measurement of the morning weight of today (step S26). The predetermined time is set to a time of an extent the weight is not greatly influenced even if exercise is carried out such as 30 minutes or one hour.
If within the predetermined time (step S26: Yes), whether or not the lightest measurement value in the morning weight of today is determined (step S27). This determination is made by comparing the morning weight data stored in the storage unit 12 as the morning weight of today and the measurement weight data in step S2.

If the value is the lightest measurement value (step S27: Yes), the measurement weight data of the previous value stored in the storage unit 12 is updated to the measurement weight data measured in step S2 to overwrite and store the data (step S28). The lighter weight measurement value is stored as the morning weight because if the weight is measured immediately after waking up and the weight is again measured after going to the restroom, the lowest weight of the day is to become the reference as the morning weight by adopting the lighter weight. The morning weight that becomes the reference then can be stabilized and the morning weight can be prevented from being registered in a state lower than the original by exercise etc. by limiting to a constant time.

The control unit 18 executes steps S24 and S25 after updating the morning weight in such a manner.

If the predetermined time has elapsed from the measurement time of the first morning weight in step S26 (step S26: No), or if the re-measured measurement weight is not the lightest (step S27: No), the control unit 18 displays a measurement weight display screen B shown in Fig. 6B on the display unit 15 (step S29).
The measurement weight display screen B displays a measurement weight title display 71 indicating that the measurement weight is being displayed, and a measurement weight display 72 displaying the measurement weight.

If determined as not the morning time (as the night time) in step S21 (step S21: No), the control unit 18 checks whether or not the night weight data of the current day is stored in the storage unit 12 (step S30).

If the night weight data is not stored (step S30: Yes), the control unit 18 newly stores the measurement weight acquired in step S2 in the storage unit 12 as the night weight (step S31). If the night weight data is stored (step S30: No), the control unit 18 updates the night weight stored in the storage unit 12 to the measurement weight acquired in step S2 to overwrite and store (step S32). The last measurement weight before going to bed is thus reliably stored as the night weight, and the night weight is stabilized.

The control unit 18 references the storage unit 12 and calculates the attainment degree (step S33). The attainment degree can be calculated by subtracting the standard daily fluctuation amount from the weight fluctuation amount of the current day obtained by subtracting the morning weight from the night weight, and further subtracting the daily target increase and decrease amount, and deciding whether such value is positive or negative or to what extent such value is positive or negative.

If the target is attained (step S34: Yes), the control unit 18 displays the target attainment result screen C shown in Fig. 6C on the display unit 15 (step S35). The target attainment result screen C includes a night weight title display 81 indicating that the weight is the night weight, a measurement weight display 82 indicating the measurement value of the night weight, a difference display 83 indicating a difference weight between the target night weight and the actual night weight, and a target attainment result display 84 indicating whether or not the target is attained. In this example, the result shows that the weight is lighter than the target night weight by 0.3 kg and that the target is attained by the difference display 83 and the target attainment result display 84.

If the target is not attained (step S34: No), the control unit 18 displays the target attainment result screen D shown in Fig. 6D on the display unit 15 (step S36).
The target attainment result screen D displays the same items as the target attainment result screen C, and thus the same reference numerals are denoted to the same items and the detailed description thereof will be omitted. In the illustrated example, the result shows that the weight is heavier than the target night weight by 0.2 kg and that the target is not attained by the difference display 83 and the target attainment result display 84.

According to the above configurations and operations, the user can check the daily target indicating to what extent the daily weight fluctuation is preferably maintained and can manage the daily weight fluctuation according to the plan to attain the target. Therefore, when dieting, whether or not the diet is progressing successfully in a very short period in units of one day can be determined.

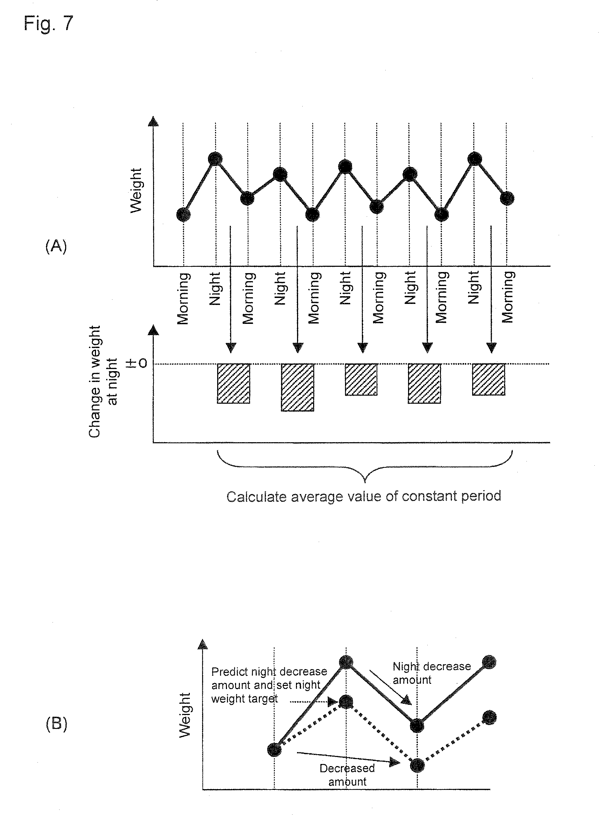

In particular, as shown in Fig. 7A, the standard daily fluctuation amount can be defined based on the actual weight increase or decrease amount by calculating the sleep weight fluctuation amount by subtracting the morning weight from the night weight, and calculating the average value of the sleep weight fluctuation amount for a plurality of days. In addition, the correct standard daily fluctuation amount can be defined regardless of whether the weight is in the increasing tendency or the decreasing tendency by using such calculation method. In other words, the weight fluctuation during sleep is only the fluctuation caused by basal metabolism, and thus a stable daily fluctuation amount can be obtained regardless of the increase or decrease in weight.

As shown in Fig. 7B, the weight of a person increases from morning to night, and decreases from night to morning. The user can manage the increase and decrease in weight in units of one day by knowing the target of to what extent to suppress the increase amount to the night weight. In other words, in the related art, if the weight measured at night is increased from the weight measured in the morning, the user can only recognize that the weight increases even though the weight is actually in the decreasing tendency. However, the user can know that the weight is in the decreasing tendency if the user is in a state the morning weight is to be decreased when measuring the weight next morning even if the night weight is increased than the morning weight by clearly indicating the target of the night weight in view of the daily fluctuation.

The morning weight can be stabilized to the weight of after going to the restroom since it can be updated to the decreased weight measurement value if the weight is decreased at the time of remeasurement within a predetermined time. In other words, the morning weight varies if the measurement of the morning weight differs between before and after going to the restroom depending on the days, but the variation of the morning weight can be suppressed by adopting the weight measurement value after going to the restroom as the morning weight as much as possible.

The night weight adopts the weight measurement value measured outside the time of setting as the morning weight, and adopts the most recent weight measurement value as the night weight every time the remeasurement is carried out, and hence the user can use the weight measurement value at the time point closest to when going to bed without any special conscious. Therefore, the night weight can be stably and accurately acquired.

In the embodiment described above, the weight and body composition meter 3 carried out all the calculations, but the server 5 may be configured to carry out various calculations and outputs.

In this case, the weight and body composition meter 3 may be configured to transmit the weight measurement value to the server 5 with the measurement date and time in step S3. When receiving the weight measurement value and the measurement date and time from the weight and body composition meter 3, the control unit 52 of the server 5 acquires the weight measurement value including the morning weight and the night weight from the weight and body composition meter 3, and executes the processes of steps S21 to S26, where storage is carried out in the storage unit 55 and the display is carried out in the display unit 54.

In this case, the control unit 52 of the server 5 is configured to transmit the information to be displayed on the screen of the display unit 54 to the weight and body composition meter 3 in steps S25, S29, S35, and S36. The weight and body composition meter 3 receives the information, and displays one of the morning measurement display screen A, the measurement weight display screen B, and the target attainment result screens C, D on the display unit 15.
In this case as well, the target of the weight increase or decrease of one day can be calculated, which can then be notified to the user. Furthermore, if the server 5 is used in such manner, the instructor or the like can check and give advice to the user.

The morning/night button may be selectively input with the operation unit 14 by the user between step S1 and step S2. In this case, whether the measurement of the morning weight or the measurement of the night weight can be determined by the input. Even the user living in a day where day and night are reversed can also make use.

If configured in such manner, the display content may be differed by the presence or absence of the pushing of the morning/night button such as displaying only the weight without displaying the target if the morning/night button is not pushed. The morning/night button thus can be prevented from being forgotten to be pushed.

The content to display in the morning measurement display screen A, the measurement weight display screen B, the target attainment result screen C, and the target attainment result screen D is not limited to the above example and may be varied. For instance, in the morning measurement display screen A, the target night weight is displayed in the target display 63, but the difference between the night weight and the morning weight may be displayed as a target value.

The target attainment result screens C, D display whether or not the target is attained by the difference display 83 and the target attainment results display 84, but this is not the sole case, and an appropriate display may be adopted such as whether or not going over the target line can be illustrated by the graph display or either the target attainment mark or the target non-attainment mark may be displayed.

The display timing is not limited to simultaneously displaying both the measurement weight of the morning and the target value and simultaneously displaying the measurement weight of the night and the target attainment result, and various timings may be used. For instance, the measurement weight may be displayed at the time point the weight measurement is completed and thereafter the display may be switched to the target display or the target attainment result display at the timing a predetermined time has elapsed or when the user gets off the upper surface cover unit 35. In this case as well, the target of one day can be checked with the morning measurement, and the result of whether or not the target is attained can be checked with the night measurement.

In the correspondence of the configuration of the present invention and the embodiment described above,
a weight management device of the present invention corresponds to the weight and body composition meter 3 or the server 5 of the embodiment, and similarly,
storage means corresponds to the storage unit 12 or the storage unit 55;
physical information acquiring means corresponds to the control unit 18 that reads out from the storage unit 12 and the control unit 52 that reads out from the storage unit 55;
output means and display means correspond to the display unit 15 or the display unit 54;
calculation means corresponds to the control unit 18 or the control unit 52;
standard daily fluctuation value acquiring means corresponds to the control unit 18 or the control unit 52 that reads out standard daily fluctuation amount in step S24;
impedance acquiring means corresponds to the impedance detection unit 20;
weight acquiring means corresponds to the load detection unit 33 or the communication unit 51;
standard daily fluctuation value corresponds to the standard daily fluctuation amount data 41;
a long term target increase and decrease amount corresponds to the long term target increase and decrease amount data 43;
a target number of days corresponds to the target attainment period data 44;
a daily target increase and decrease amount corresponds to the daily target increase and decrease amount data 45;
a daily target value corresponds to the target value of the night weight shown in the target display 63;
a determination result corresponds to the result to display on the target attainment result display 84;
a waking up side weight measurement value updating process corresponds to steps S26 to S28;
a going to bed side weight measurement value updating process corresponds to steps S30 to S32;
a target attainment determination process corresponds to step S34;
a first timing corresponds to the morning time;
a second timing corresponds to the night time;
a waking up side weight measurement value corresponds to the morning weight;
a going to bed side weight measurement value corresponds to the night weight; and
the physical information corresponds to the sex, age, height, and weight.
However, the present invention is not limited to only the configuration of the embodiment described above, and a great number of embodiments may be contrived.

### INDUSTRIAL APPLICABILITY

The present invention can be used in the field of managing the weight of a living body, and can also be used in managing the weight of not only humans but also of animals.

### DESCRIPTION OF SYMBOLS

- 3: weight and body composition meter
- 5: server
- 12: storage unit
- 15: display unit
- 18: control unit
- 20: impedance detection unit
- 33: load detection unit
- 41: standard daily fluctuation amount data
- 43: long term target increase and decrease amount data
- 44: target attainment period data
- 45: daily target increase and decrease amount data
- 51: communication unit
- 52: control unit
- 54: display unit
- 55: storage unit
- 63: target display
- 84: target attainment result display

## Claims

1. A weight management device comprising:
weight acquiring means for acquiring a weight measurement value of a user;
standard daily fluctuation amount acquiring means for acquiring a standard daily fluctuation amount indicating a standard daily fluctuation of the weight of the user; and
calculation means for calculating a daily target value that becomes a target when measuring a going to bed side weight measurement value at a second timing on the going to bed side with respect to a waking up side weight measurement value measured at a first timing on a waking up side from waking up until going to bed of the user based on the standard daily fluctuation amount.

2. The weight management device according to claim 1, further comprising:
output means for outputting the daily target value; wherein
the calculation means outputs the daily target value to the output means when acquiring the waking up side weight measurement value from the weight acquiring means.

3. The weight management device according to claim 2, further comprising:
storage means for storing the waking up side weight measurement value; wherein
the calculation means acquires the going to bed side weight measurement value from the weight acquiring means, executes a target attainment determination process of determining whether or not the daily target is attained based on the going to bed side weight measurement value, the daily target value, and the waking up side weight measurement value, and outputs the determination result of the target attainment determination process to the output means.

4. The weight management device according claim 1, wherein the calculation means is adapted to calculate the standard daily fluctuation amount based on a plurality of sleep weight fluctuation amount that is a difference between the going to bed side weight measurement value measured over plural days in the past and the waking up side weight measurement value of a next day.

5. The weight management device according to claim 1, further comprising:
basal metabolism amount acquiring means for acquiring a basal metabolism amount of the user based on physical information related to the body of the user and an impedance of the body of the user; wherein the calculation means is configured to calculate the standard daily fluctuation amount based on the basal metabolism amount.

6. The weight management device according to claim 1, wherein
the calculation means may have a configuration to execute a waking up side weight measurement value updating process of updating the waking up side weight measurement value to a remeasured weight measurement value when the weight is remeasured within a predetermined time from the first timing at which the waking up side weight measurement value is measured and the remeasured weight measurement value is lower than the waking up side weight measurement value.

7. The weight management device according to claim 1, wherein
the calculation means is configured to execute a going to bed side weight measurement value determination process of adopting the weight measurement value measured last within a predetermined time as the going to bed side weight measurement value when the weight is measured over plural times within the predetermined time of recognized as the going to bed side weight measurement value.

8. The weight management device according to claim 1, wherein
the calculation means,
calculate a daily target increase and decrease amount by dividing a long term target increase and decrease amount that is a target weight increase and decrease amount by a target number of days until attaining the target, and
defines the daily target value based on the daily target increase and decrease amount and the standard daily fluctuation amount.

9. The weight management device according to claim 1, wherein
the first timing is from when the user wakes up until before breakfast, and the second timing is from after dinner until before the user goes to bed.

10. A weight management device comprising:
weight acquiring means for acquiring a weight measurement value of a user,
information processing means for performing information processing, and
display means for performing display of the screen, wherein
the information processing means displays on the display means a waking up side weight display screen if the weight measurement value of the user acquired by the weight acquiring means is a waking up side weight measurement value measured at a first timing on the waking up side from when the user wakes up until breakfast, and displays on the display means a going to bed side weight display screen different from the waking up side weight display screen if the weight measurement value is a going to bed side weight measurement value measured at the second timing on the going to bed side.

11. A weight management method comprising the steps of:
acquiring a weight, measurement value of a user with weight acquiring means;
acquiring a standard daily fluctuation amount indicating a standard daily fluctuation of a weight of the user with standard daily fluctuation amount acquiring means; and
calculating a daily target value to become a target when measuring a going to bed side weight measurement value at a second timing on a going to bed side with respect to a waking up side weight measurement value measured at a first timing on a waking up side from when the user wakes up until going to bed based on the standard daily fluctuation amount with calculation means.

12. A weight measurement program for causing a computer to function as:
weight acquiring means for acquiring a weight measurement value of a user;
standard daily fluctuation amount acquiring means for acquiring a standard daily fluctuation amount indicating a standard daily fluctuation of the weight of the user; and
calculation means for calculating a daily target value to become a target when measuring a going to bed side weight measurement value at a second timing on a going to bed side with respect to a waking up side weight measurement value measured at a first timing on a waking up side from when the user wakes up until going to bed based on the standard daily fluctuation amount.
